# EUROPÄISCHE PATENTSCHRIFT

(11) **EP 2 922 483 B1**
(45) Veröffentlichungstag und Bekanntmachung des Hinweises auf die Patenterteilung: **01.01.2020**
(21) Anmeldenummer: 13811133.1
(22) Anmeldetag: 18.11.2013
(51) Int. Cl.: A61B 17/34, A61B 17/00

(54) **TROKARVORRICHTUNG UND DEREN VERWENDUNG**
TROCAR DEVICE AND USE THEREOF
DISPOSITIF FORMANT TROCART ET SON UTILISATION

(30) Priorität: 20.11.2012 DE 102012111192
(43) Veröffentlichungstag der Anmeldung: 30.09.2015
(73) Patentinhaber: Kreuz, Gerold, 02997 Wittichenau (DE); Koch, Heinrich, 83301 Traunreut-Matzing (DE)
(72) Erfinder: KREUZ, Gerold, 02997 Wittichenau (DE); KOCH, Heinrich, 83301 Traunrreut-Matzing (DE)
(74) Vertreter: Kailuweit & Uhlemann Patentanwälte Partnerschaft mbB
(86) Internationale Anmeldenummer: PCT/EP2013/074063
(87) Internationale Veröffentlichungsnummer: WO 2014/079807

(56) Entgegenhaltungen:
- EP-A1- 0 537 758
- EP-A1- 1 774 918
- US-A- 3 713 447
- US-A- 3 713 447
- US-A1- 2003 060 770
- US-A1- 2009 306 586
- US-A1- 2011 144 440
- US-A1- 2011 144 447
- US-A1- 2011 218 565

## Beschreibung

Die Erfindung betrifft eine Trokarhülse bzw. Trokar sowie einen Trokardorn für minimal invasive / endoskopische chirurgische Eingriffe an Mensch und Tier.

Die ersten Laparoskopien wurden bereits 1944 von Hans Frangenheim und Raoul Palmer publiziert. Als Pionier der laparoskopischen bzw. pelviskopischen Operationen darf jedoch Kurt Semm gelten, der mit seinen damals spektakulären Eingriffen und Erfindungen die Chirurgie revolutioniert und gegen viele Anfeindungen ab Mitte der siebziger Jahre des vorigen Jahrhunderts etabliert hat.

In der endoskopischen und laparoskopischen Chirurgie wird mittlerweile die überwiegende Anzahl der Eingriffe minimal invasiv durchgeführt. Dies betrifft alle operativ tätigen Fachgebiete. Der Begriff minimal invasiv oder Schlüsselloch Chirurgie bezeichnet Operationstechniken über kleinste Zugänge mit speziell dafür entwickelten Instrumenten und Kameras. Trokare sind Standardinstrumente in der minimal invasiven Chirurgie, deren Anwendung spezielle Besonderheiten und Risiken beinhaltet.

Der Trokar ist ein Instrument, mit dessen Hilfe in der Chirurgie scharf oder stumpf, nach vorheriger Hautincission, eine Öffnung als Zugang zu einer Körperhöhle geschaffen wird. Dabei wird zuvor beim Bauchraum dieser in der Regel mit vorgewärmtem CO₂ aufgebläht, um bessere Sichtverhältnisse zu schaffen. Durch eine Trokarhülse wird diese offen gehalten. Es handelt sich üblicherweise um einen Trokardorn bzw. Stift, der in der Trokarhülse mit einem Innendurchmesser von z.B. 3,0 - 12 mm sitzt und dessen Spitze die Öffnung der Trokarhülse verschließt. Der Trokar wird z.B. durch die Bauchdecke oder die Nabelgrube in den Bauchraum eingeführt. Der Operateur hat dann nach dem Herausziehen des Trokardorns aus der Trokarhülse die Möglichkeit, mit einer Optik (Endoskop) durch die Trokarhülse in den Bauchraum zu schauen oder mit Greif-, Schneide- und anderen Instrumenten innerhalb des Bauchraumes minimal invasiv zu operieren. (Die Durchmesser der Instrumente sind genormt, so dass Trokare und Instrumente verschiedenster Hersteller zueinander passen). Dies kann durch einen mit der Trokarhülse verbundenen Trokarkopf geschehen, der einen gasdichten Port für die vorgenannten Instrumente aufweist. Moderne Trokar Systeme sind entweder aus chirurgischem Stahl oder medizinischem Kunststoff. Sie werden sowohl als wiederverwendbare oder Einweginstrumente angeboten. Wobei der Trend mehr und mehr zu Einweg geht, da die Aufbereitungsrichtlinien stets strenger werden und Trokare aufgrund der vorhandenen Hohlräume als sog. semikritische Instrumente für die Wiederaufbereitung eingestuft werden. Der Obturator oder Trokardorn ist an die Trokarhülse angepasst und kann sowohl eine schneidende als auch eine stumpfe Spitze aufweisen. Daneben gibt es noch sog. Sicherheitstrokare, bei denen die schneidende Spitze mit einer Klinge versehen ist, die nach dem Durchdringen der Bauchdecke zurückfährt, um eine Verletzung von inneren Organen zu vermeiden. Die Trokarhülsen können einen Ventilmechanismus oder Anschluss aufweisen, welcher zum Absaugen, Insufflieren etc. genutzt wird. Gemäß dem Stand der Technik sind Trokarhülsen starr und biegefest ausgeprägt, um die Führungseigenschaften für Instrumente bei den während der Operation nötigen Manipulationen mit den Instrumenten beizubehalten.

Allen minimal invasiven Eingriffen vorrangig im Bereich der Bauchhöhle und der Pelvis ist die Benutzung von Trokaren, zur Schaffung des Zugangs in die Körperhöhle und zum Einbringen von Optik und Instrumenten gemein. Trokare und ihre tägliche Anwendung sind im Bereich der Gynäkologie, Urologie und Chirurgie selbstverständlich geworden.

Trokar Systeme sind in verschiedensten Ausführungen sowohl als Mehrweg-, als auch als Einwegsysteme bekannt. Sie bestehen in der Regel aus einer Trokarhülse und einem innerhalb der Trokarhülse beweglichen Trokardorn, welcher an seinem distalen Ende oftmals mit einer scharfen Schneide zur Durchdringung von Gewebe ausgestattet ist.

Druckschriften wie z.B. US 2005/0251190 oder US 2007/008827 beschreiben gasdichte Zugangswege unter Verwendung von Dichtungen und Anschluss-Systemen. Dichtungssysteme sind unter anderem auch aus den US 4,943,280; US 4,655,752; US 4,978,341; EP 0 567 141 bekannt. Letztgenannte beschreibt ein Ventilsystem, das das Einführen chirurgischer Instrumente in den Körper eines Patienten insbesondere über ein Trokarsystem erlaubt. Ein gasdichter Abschluss ist für Instrumente unterschiedlicher Größe gewährleistet.

Mehr als 50% der Komplikationen erfolgen intraoperativ und somit ist schnelles Handeln des Operateurs zur Abwendung lebensbedrohlicher Situationen gefordert. Dabei kommt es gerade beim endoskopischen Operieren vor, dass in diesen außerplanmäßigen Situationen, schnell und häufig die über den Trokar eingebrachten Instrumente gewechselt werden müssen. Sehr häufig besitzen die heute im Markt befindlichen Trokare nicht die geforderte Haltefunktion z. B. in der Bauchdecke und rutschen mit dem Instrument heraus. Dadurch entstehen schnell unbeherrschbare Zustände, weil eine (zeit)aufwändige Neuplatzierung vonnöten ist. Dieser Stressfaktor für das gesamte OP Team kann zu weitergehenden Komplikationen führen. Wünschenswert wäre somit ein Trokar, der sicher in allen Situationen hält, der atraumatisch ist, der eine günstige Kosten- Nutzen- Relation aufweist und der last but not least einfach und unkompliziert "dem chirurgischen Denken" entsprechend platziert und entfernt werden kann.

Weiterhin bekannt sind auch Trokarsysteme wie das Kii Balloon Blunt System (Applied Medical), welches einen aufblasbaren Ballon im vorderen Bereich der Trokarhülse aufweist. Nach dem Einführen des Trokars wird dabei Luft in den Ballon geblasen, wodurch dieser sich ausdehnt und ein Herausrutschen des Trokars verhindern soll. Nachteilig bei dem System ist vor allem die Anordnung des Ballons an der Außenseite des Trokars, wodurch der Ballon mit durch den Einstichkanal geführt werden muss, was sich in der praktischen Anwendung als nachteilig herausgestellt hat. Zudem ist ein zusätzlicher Anschluss für die Luftzufuhr vorgesehen, was in der Regel die Handhabung erschwert und die Zeit zur Einführung des Trokars für den Operateur verlängert. Weiterhin besteht die Gefahr, dass der Ballon aufgrund der Ausführungen unter der Operation Beschädigungen erfährt, was zu einer fehlenden Funktionalität des Ballons und der Gefahr des Herausrutschens des Trokars unter der Operation führen kann.

US 2011/144447 A1 offenbart ein Verfahren und eine Vorrichtung zur Schaffung eines Zugangs, wobei die offenbarte Trokarhülse mehrstückig ausgebildet ist und eine Ankervorrichtung aufweist. US 2011/144440 A1 offenbart ebenfalls ein Verfahren und eine Vorrichtung zur Schaffung eines chirurgischen Zugangs mit einer mehrstückigen Trokarhülse mit einer Ankervorrichtung.

Der vorliegenden Erfindung liegt die Aufgabe zugrunde, eine verbesserte Trokarhülse sowie einen verbesserten Trokardorn für die Anwendung an Mensch und Tier bereitzustellen.

Diese Aufgabe wird durch einen Trokar nach Anspruch 1, ein System nach Anspruch 9 und ein Verfahren nach Anspruch 10 gelöst. Vorteilhafte Ausgestaltungen sind in den abhängigen Ansprüchen angegeben.

Ein Aspekt der Erfindung betrifft einen Trokar umfassend eine Trokarhülse sowie einen Trokarkopf. Ein erfindungsgemäßer Aspekt der Erfindung betrifft eine Trokarhülse, welche einstückig ausgebildet ist, umfassend einen ersten und einen zweiten Bereich, wobei der erste Bereich zur Positionierung innerhalb eines Körpers ausgebildet ist und ein Haltemittel zur Vermeidung des Herausrutschens der erfindungsgemäßen Trokarhülse aus dem Körper aufweist, wobei das Haltemittel stegförmig ausgebildete Bereiche aufweist, welche durch Öffnungen in der Trokarhülse voneinander beabstandet sind. Die stegförmigen Bereiche weisen dabei einen gebeugten Ausgangszustand auf, sodass die stegförmigen Bereiche einen aufgepilzten Zustand aufweisen. Die stegförmigen Bereiche sind dabei derart ausgebildet, dass diese von einem gebeugten Ausgangszustand in einen getreckten Zustand übergehen können. Im gestreckten Zustand sind die stegförmigen Bereiche im Wesentlichen planar ausgebildet, und die Oberfläche der Trokarhülse weist eine im Wesentlichen geschlossene Oberfläche auf.

Die erfindungsgemäße Trokarhülse weist zudem einen zweiten Bereich auf, welcher zur Aufnahme eines Trokardorns ausgebildet ist.

Unter einer einstückigen Trokarhülse wird im Sinne der Erfindung eine Trokarhülse verstanden, welche aus einem Werkstück ausgebildet ist. Diese kann beispielsweise mittels Spritzgussverfahren hergestellt sein.

In der Ausführungsform der Erfindung sind die stegförmigen Bereiche aus einem Formgedächtnismaterial ausgebildet bzw. in einer nicht erfindungsgemäßen Ausführungsform werden diese so gefertigt, dass sie nach dem Strecken wieder in ihren Ausgangszustand zurückkehren. Unter Formgedächtnismaterialien und/oder Fertigungstechniken werden Materialien und/oder Herstellungstechniken verstanden, welche einen Formgedächtniseffekt (shape-memory effect) aufweisen oder schaffen und sich an ihre frühere äußere Form trotz einer zwischenzeitlichen starken Umformung scheinbar "erinnern" können. Solche Formgedächtnismaterialien bzw. Herstellungstechniken sind beispielsweise Formgedächtnislegierungen und Formgedächtnispolymere und/oder Herstellungstechniken. Dabei sind die stegförmigen Bereiche durch das Formgedächtnismaterial oder die Form so ausgebildet, dass diese einen gekrümmten Ausgangszustand aufweisen und somit eine Annäherung des ersten Bereichs der Trokarhülse an den zweiten Bereich der Trokarhülse bewirken. Dadurch kommt es zu einer Aufpilzung der stegförmigen Bereiche im ersten Bereich, wodurch ein Herausrutschen aus dem Körper vermieden wird.

In einer Ausführungsform der Erfindung sind die stegförmigen Bereiche aus einem Formgedächtnismaterial ausgebildet, wobei das Formgedächtnismaterial ein Kunststoff ist.

In einer weiteren Ausführungsform der Erfindung sind die stegförmigen Bereiche halbschalenförmig ausgebildet. Die so gestalteten halbschalenförmigen Bereiche weisen mit der gerundeten Seite in Richtung Innenseite der Trokarhülse. In Richtung Außenseite weisen die stegförmigen Bereiche eine im Wesentlichen ebene Fläche auf, welche aufgrund der zylinderförmigen Ausgestaltung der Trokarhülse eine Krümmung aufweist. Im gestreckten Zustand sind die stegförmigen Bereiche mit dem ersten Bereich fluchtend ausgerichtet. Durch die halbschalenförmige Ausgestaltung der stegförmigen Bereiche sind diese hinreichend stabil, um eine wiederholte Streckung und Beugung zu gewährleisten. Aufgrund der im Wesentlichen ebenen Oberfläche wird zudem eine Verletzungsgefahr im Randbereich des Einstichs etwa durch Grate, Scharniere oder dergleichen vermieden. Vorzugsweise passt sich die halbschalenförmige Innenseite der stegförmigen Bereiche während der Streckung in eine am Trokardorn angeformte Ausnehmung ein, sodass die stegförmigen Bereiche und die Ausnehmung in Eingriff stehen. Die halbschalenförmige Ausgestaltung der stegförmigen Bereiche gewährleistet dabei in einfacher Weise dem Operateur eine einfache und kraftsparende Manipulation der Trokarhülse ohne dabei die Führungseigenschaften für die einzubringenden Instrumente zu verlieren und ohne an den dem Gewebe bzw. Peritoneum anliegenden Stellen Verletzungen hervorzurufen.

In einer Ausführungsform der Erfindung ist der zweite Bereich biegesteif ausgebildet.

In einer weiteren Ausführungsform der Erfindung weist zumindest der zweite Bereich, vorzugsweise die gesamte Trokarhülse, einen im Wesentlichen gleichbleibenden Querschnitt auf, welcher kreisförmig, oval oder rund ausgebildet ist.

In einer weiteren Ausführungsform der Erfindung weist zumindest der zweite Bereich, vorzugsweise die gesamte Trokarhülse, einen im Wesentlichen gleichbleibenden Querschnitt auf, welcher kreisförmig ausgebildet ist. Ein kreisförmiger Querschnitt bietet den Vorteil der Stabilisierung während der Manipulation mit Instrumenten.

In einer weiteren Ausführungsform der Erfindung weist zumindest der zweite Bereich, vorzugsweise die gesamte Trokarhülse, einen im Wesentlichen gleichbleibenden Querschnitt auf, welcher oval ausgebildet ist. Dabei erlaubt ein ovaler Querschnitt mehr Bewegungsspielraum für die Instrumente, ohne dass die Toleranzen zu eng gelegt werden müssen.

In einer weiteren Ausführungsform der Erfindung weist die Trokarhülse außen Markierungen zum Ablesen ihrer eingeführten Länge auf. Durch die außen an der Trokarhülse angebrachten Markierungen kann die eingeführte Länge der Trokarhülse bestimmt werden, wodurch mögliche Gewebe- bzw. Organverletzungen beim Patienten vermieden werden.

In einer weiteren Ausführungsform der Erfindung weist die Trokarhülse eine Stoppvorrichtung auf. Diese Stoppvorrichtung dient dabei der Verhinderung eines selbstständig infolge des durch Manipulationen mit Instrumenten verursachten Hineinrutschens in den Bauchraum bzw. die Körperhöhle.

In einer weiteren Ausführungsform der Erfindung ist die Trokarhülse mit Ausnahme der stegförmigen Bereiche biegefest ausgebildet.

In einer weiteren Ausführungsform der Erfindung weist die Trokarhülse einen Innendurchmesser von 3 - 15 mm auf. In Abhängigkeit des jeweiligen Verwendungszwecks weist die Trokarhülse einen Innendurchmesser von 5 - 5,5 mm oder 7 - 7,5 mm oder 10 - 10,5 mm oder 12 -12,5 mm auf.

In einer weiteren Ausführungsform der Erfindung weist eine Trokarhülse über einen zumindest wesentlichen Teil der Gesamtlänge eine Wandstärke von 0,2 - 2 mm. In Abhängigkeit des jeweiligen Verwendungszwecks weist die Trokarhülse eine Wandstärke von 0,5 - 1,0 mm oder von 0,8 - 1,2 mm oder von 1,0 mm oder 1,5 mm auf.

In einer weiteren Ausführungsform der Erfindung weist die Trokarhülse eine Gesamtlänge von 70 - 230 mm auf. In Abhängigkeit des jeweiligen Verwendungszwecks weist die Trokarhülse eine Gesamtlänge von 70 - 80 mm oder 80 - 90 mm oder 110 - 120 mm oder 150 - 160 mm oder 160 - 170 mm auf. Die zuvor genannten Maße sind besonders für die Verwendung über einen Zugang durch die Bauchdecke in den Bauchraum bzw. die Pelvis geeignet.

In einer weiteren Ausführungsform der Erfindung weist die Trokarhülse im ersten Bereich eine Stirnseite auf, welche konifiziert ausgebildet ist.

In der Ausführungsform der Erfindung weist die Trokarhülse im Inneren zumindest ein Mittel zur lösbaren Anordnung des Trokardorns auf. Dabei wird der Trokardorn in die Trokarhülse eingeführt und mittels des zumindest einen Mittels zur Anordnung in der Trokarhülse angeordnet. Das Mittel zur Anordnung kann dabei beispielsweise als Mittel zur formschlüssigen Verbindung ausgebildet sein. Beispielsweise kann die formschlüssige Verbindung mittels einer Rastverbindung aus Rastnut und Rastnase erfolgen. Dabei kann die Trokarhülse im Inneren als ein Mittel zur Anordnung eine Rastnut oder Rastnase aufweisen. Es ist auch denkbar, dass zur Anordnung mehrere Mittel zur Anordnung genutzt werden.

In einer Ausführungsform der Erfindung ist der Trokar einstückig ausgeführt, wobei der Trokarkopf an einer erfindungsgemäßen Trokarhülse angeformt ist.

In einer Ausführungsform der Erfindung ist der Trokar zweistückig ausgeführt. Dabei sind die Trokarhülse und der Trokarkopf flüssig- und gasdicht unter Zwischenlage einer Dichtung und/oder einer formschlüssigen Verbindung miteinander verbunden. Der Trokarkopf ist in der Regel mehrfach verwendbar, wobei nur die Trokarhülse als Wegwerfartikel aus einem geeigneten Material gefertigt werden müsste.

In einer Ausführungsform der Erfindung ist der Trokar zweistückig ausgeführt, wobei die Trokarhülse und der Trokarkopf lösbar miteinander verbunden sind.

In einer Ausführungsform der Erfindung ist innerhalb des Trokarkopfes eine Dichtung lösbar angeordnet.

In einer Ausführungsform der Erfindung umfasst der Trokarkopf mindestens ein gasdichtes Ventil und/oder mindestens einen Auslass für Gase und/oder Flüssigkeiten. Das Ventil und/oder der Auslass werden dabei zur Insufflation von Gas oder dem Ablassen von bei der Koagulation entstehendem, die Sicht behinderndem Rauch verwendet.

Ein weiterer Aspekt betrifft einen Trokardorn, mit einem ersten und einen zweiten Bereich, wobei der zweite Bereich gegenüber dem ersten Bereich verjüngt ausgebildet ist und wobei der erste Bereich eine Stirnseite aufweist. Die Stirnseite kann dabei konisch spitz zulaufen oder stumpf ausgebildet sein. Zu Beginn der Operation wird ein Trokardorn verwendet, der eine konisch spitz zulaufende Ausgestaltung aufweist, die derart bzw. in Länge und Durchmesser an den zweiten Bereich der Trokarhülse angepasst ist, dass sie in der Konifizierung dem ersten Bereich der Trokarhülse zum besseren Perforieren der Bauchwand im Wesentlichen entspricht. Der erste Bereich des Trokardorns ist so ausgeformt, dass es dem Operateur eine beim Einstechen und Einführen in die Körperhöhle möglichst belastungs- und kraftsparende ergonomische Vorgehensweise ermöglicht.

Entsprechend einem weiteren Aspekt weist der Trokardorn Ausnehmungen auf, welche zur Aufnahme der stegförmigen Bereiche einer Trokarhülse ausgebildet sind. Vorzugsweise sind die Ausnehmungen radial entlang des Trokardorns angeordnet, wobei die Anzahl der Ausnehmungen der Anzahl der stegförmigen Bereiche der Trokarhülse entspricht. Beispielsweise sind 1 bis 8, vorzugsweise 2 bis 6, besonders bevorzugt 2, 4 oder 6 Ausnehmungen entlang des Trokardorns angeordnet. Vorzugsweise weisen die Ausnehmungen einen halbkreisförmigen Querschnitt auf.

In einer Ausführungsform der Erfindung ist der Trokardorn einstückig ausgeführt.

In einem weiteren Aspekt, welcher nicht Teil der vorliegenden Erfindung ist, ist der Trokardorn zweistückig ausgebildet, wobei der erste und der zweite Bereich lösbar verbunden sind. Dadurch kann der erste Bereich oder der zweite Bereich wieder verwendet werden, während der respektiv andere Bereich als Einweg-Produkt ausgestaltet ist. Dadurch lassen sich erhöhte Aufwendungen durch Sterilisation vermeiden, wenn nur bestimmte Teile des Trokardorns beispielsweise aus einem Metall oder -legierung ausgebildet werden müssen.

In einer weiteren Ausführungsform der Erfindung weist der Trokardorn zumindest ein Mittel zur lösbaren Anordnung des Trokardorns in der erfindungsgemäßen Trokarhülse auf. Dabei wird der Trokardorn in die Trokarhülse eingeführt und mittels des zumindest einen Mittels zur Anordnung in der Trokarhülse angeordnet. Das Mittel zur Anordnung kann dabei beispielsweise als Mittel zur formschlüssigen Verbindung ausgebildet sein. Beispielsweise kann die formschlüssige Verbindung mittels einer Rastverbindung aus Rastnut und Rastnase erfolgen. Dabei kann der Trokardorn als ein Mittel zur Anordnung eine Rastnut oder Rastnase aufweisen. Es ist auch denkbar, dass zur Anordnung mehrere Mittel zur Anordnung genutzt werden können.

Ein weiterer nicht erfindungsgemäßer Aspekt betrifft ein Kit umfassend eine Trokarhülse sowie zumindest einen Trokardorn. Vorteilhaft umfasst das Kit eine Trokarhülse sowie einen Trokardorn mit spitz zulaufender konischer Stirnseite sowie einen zweiten Trokardorn mit stumpfer abgerundeter Stirnseite.

Ein weiterer nicht erfindungsgemäßer Aspekt betrifft ein System umfassend einen Trokar sowie zumindest einen Trokardorn. Vorteilhaft umfasst das System eine Trokarhülse sowie einen Trokardorn mit spitz zulaufender konischer Stirnseite sowie einen zweiten Trokardorn mit stumpfer abgerundeter Stirnseite.

Ein weiteres Beispiel, welches nicht Teil der vorliegenden Erfindung ist, betrifft die Verwendung eines Kits zur endoskopischen Chirurgie und/oder Diagnostik.

Ein weiteres Beispiel, welches nicht Teil der vorliegenden Erfindung ist, betrifft die Verwendung eines Systems zur endoskopischen Chirurgie und/oder Diagnostik.

Ein weiterer Aspekt der Erfindung betrifft ein Verfahren zum Spannen stegförmiger Bereiche einer Trokarhülse des erfindungsgemäßen Trokars außerhalb des menschlichen oder tierischen Körpers umfassend die Schritte:
- Einführen eines Trokardorns in eine Trokarhülse, wobei die Stirnseite des Trokardorns im Inneren an eine Stirnseite der Trokarhülse anschlägt,
- Anordnen des Trokardorns in der Trokarhülse durch zumindest eine lösbare Anordnung, wobei ein im Inneren der Trokarhülse angeordnetes Mittel zur lösbaren Anordnung mit einem am Trokardorn angeordneten Mittel zur lösbaren Anordnung in Eingriff gelangt, sodass der Trokardorn in distaler Richtung bewegt wird, sodass die stegförmigen Bereiche durch die distale Bewegung des Trokardorns von einem gebeugten Ausgangszustand in eine gestreckte Position überführt werden, wodurch die Trokarhülse eine im Wesentlichen geschlossene Oberfläche aufweist.

In einer Ausführungsform der Erfindung erfolgt die lösbare Anordnung durch eine Verrastung einer Rastnut und einer Rastnase.

In einer Ausführungsform der Erfindung erfolgt Verrasten von Rastnut und Rastnase durch eine Drehbewegung des Trokardorns. Bei der Drehung des Trokardorns gelangen Rastnase und Rastnut in Eingriff. Die Verrastung des Trokardorns kann durch Drehung des Trokardorns in Gegenrichtung wieder gelöst werden.

In einer Ausführungsform der Erfindung erfolgt die lösbare Anordnung durch eine formschlüssige Verbindung. Diese kann beispielsweise als Klemm-, Steck- oder Stiftverbindung ausgebildet sein.

Die Aufpilzung der stegförmigen Bereiche im ersten Bereich der Trokarhülse verhindert ein komplettes oder partielles Herausrutschen der Trokarhülse unter der Operation. Infolge der Aufpilzung ist die Auflagefläche, welche durch die aufgepilzten stegförmigen Bereiche ausgebildet wird, derart vergrößert, dass diese an der Bauchinnenwand anliegend ein Herausrutschen sicher verhindern. Die im "Ruhezustand" bzw. "Auslieferungszustand" aufgepilzten stegförmigen Bereiche werden durch Einführen und Verrasten oder Drehen des Trokardorns gestreckt und auf den Außendurchmesser der Trokarhülse angepasst. Die Abmessungen sind so berechnet, dass es zu keinem Überstrecken und dadurch Beschädigen der stegförmigen Bereiche kommen kann. Es ist aber durchaus denkbar, dass das System bereits im "gestreckten" Zustand ausgeliefert wird, wobei im Inneren der Trokarhülse der Trokardorn angeordnet ist.

Im Rahmen des beispielhaften Einsatzes in chirurgischen Anwendungen erfolgt danach das Entfernen der Trokarhülse aus dem Körper, wobei der Trokardorn erneut in die Trokarhülse eingeführt und verrastet wird, wodurch die stegförmigen Bereiche gestreckt bleiben und ein einfaches Entfernen der Trokarhülse gewährleistet wird.

Durch die erneute Einführung und Verrasten oder Verdrehen des Trokardorns erfolgt eine Streckung der stegförmigen Bereiche im ersten Bereich der Trokarhülse, wodurch die Aufpilzung aufgehoben wird und nunmehr ein einfaches Entfernen der Trokarhülse ermöglicht wird.

Ein beispielhafter weiterer Aspekt betrifft ebenfalls ein Verfahren zur Vorbereitung einer Behandlung, zur Behandlung bzw. Diagnostik in der endoskopischen Chirurgie an Mensch oder Tier. Ein derartiges Verfahren umfasst die Bereitstellung einer Trokarhülse bzw. eines erfindungsgemäßen Trokars, eines Obturators/Trokardorns und ggf. die entsprechende Bedienung. Dabei wird der Trokardorn in die erfindungsgemäße Trokarhülse eingeführt, wodurch die aufgepilzten stegförmigen Bereiche gestreckt werden. Danach erfolgt der Einstich des Trokars im Operationsbereich. Anschließend wird der Trokardorn aus der Trokarhülse entfernt, wodurch die Streckung der stegförmigen Bereiche aufgehoben wird und diese sich nunmehr im Inneren des Körpers aufpilzen. Nach erfolgter Operation wird der Trokardorn erneut eingeführt und die stegförmigen Bereiche wiederum gesteckt. Danach kann der Trokar vollständig ohne größeren Aufwand entfernt werden.

Die vorliegende Erfindung ermöglicht einen sicheren, sterilen Zugang zum Bauchraum. Weiterhin verhindert sie das ungewollte Herausrutschen des Trokars bzw. der Trokarhülse aufgrund ungenügender eigenständiger Haltefunktion in der Bauchdecke, wie mittels Vergleichsversuchen festgestellt werden konnte. Dabei stellte sich heraus, dass die im Trokarkopf eingebrachte(n) Dichtung(en), welche die durchaus gewollte Gasdichtheit gewährleisten sollen, den eingebrachten Instrumenten einen Reibungswiderstand entgegensetzen, der für einen sog. Mitnahmeeffekt sorgt, und die Trokarhülse ganz oder teilweise in die Bauchdecke zurückzieht bzw. diese ganz herauszieht. Ein weiterer Vorteil der vorliegenden Erfindung ist es, ein chirurgisches Instrument anzugeben, das nach Beendigung des Eingriffes einen sicheren Verschluss der Eintrittsöffnung erlaubt.

Der Einstich erfolgt vorzugsweise durch die Bauchwand bis das distale Ende des Trokars bzw. die nach dem Zurückziehen des Trokardorns verbleibende Trokarhülse etwas über das Peritoneum in den Bauchraum ragt, wobei das Haltemittel, welches beispielsweise als speziell geformte Aufpilzung ausgebildet ist, zum einen ein Zurückrutschen bzw. ein Herausrutschen innerhalb des Stichkanales bzw. aus dem Stichkanal verhindert. Die spezielle Aufpilzung ist formfest, was heißt, dass nach dem Öffnen der Aufpilzung ein formfester Zustand erreicht wird. Formfest heißt zudem, dass die Trokarhülse beim bestimmungsgemäßen Gebrauch im Wesentlichen ihre Form und Führungseigenschaften für Instrumente etc. beibehält. Dazu kann das Haltemittel beispielsweise aus einem Formgedächtnismaterial ausgebildet sein.

Bevorzugte Weiterbildungen der Erfindung ergeben sich aus den Kombinationen der Ansprüche oder einzelner Merkmale davon.

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

Es zeigen
Fig. 1 eine schematische Querschnittsdarstellung einer erfindungsgemäßen Trokarhülse mit einer Aufpilzung der stegförmigen Elemente im ersten Bereich der Trokarhülse,
Fig. 2 eine schematische Darstellung einer erfindungsgemäßen Trokarhülse mit einem eingeführten Trokardorn mit einer Aufpilzung der stegförmigen Elemente im ersten Bereich der Trokarhülse,
Fig. 3 eine schematische Querschnittsdarstellung einer erfindungsgemäßen Trokarhülse mit einem eingeführten Trokardorn mit einer Aufpilzung der stegförmigen Elemente im ersten Bereich der Trokarhülse, Fig. 4 eine weitere schematische Darstellung einer erfindungsgemäßen Trokarhülse mit eingeführtem Trokardorn und gestreckten stegförmigen Bereichen,
Fig. 5 eine schematische Darstellung einer erfindungsgemäßen Trokarhülse mit eingeführtem Trokardorn und
Fig. 6 eine schematische Darstellung eines Trokardorns.

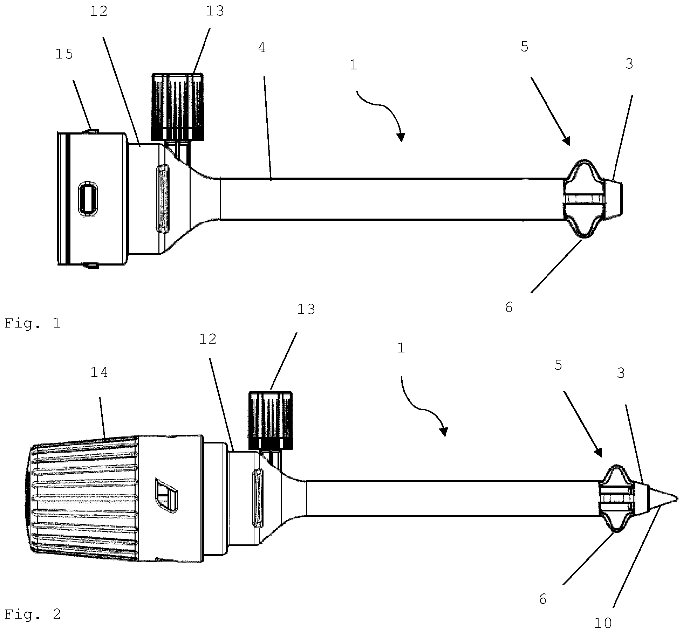

In einem ersten Ausführungsbeispiel ist in Fig. 1 schematisch eine erfindungsgemäße Trokarhülse dargestellt. Die Trokarhülse 1 weist dabei einen ersten und einen zweiten Bereich 3,4 auf. Der erste Bereich 3 weist an seinem Ende eine Stirnseite auf, welche konisch ausgeführt ist. Im ersten Bereich 3 weist die Trokarhülse ein Haltemittel 5, welches beispielsweise als stegförmige Bereiche 6 ausgebildet ist, auf. Diese stegförmigen Bereich können dabei etwa aus einem Formgedächtnismaterial, beispielsweise einem Formgedächtniskunststoff oder durch spezielle Fertigungstechniken, ausgebildet sein und beispielsweise eine halbschalenförmige Ausgestaltung in Richtung des Inneren der Trokarhülse 1 aufweisen. Dabei sind die stegförmigen Bereiche 6 im Ausgangszustand in einer gekrümmten Position, sodass eine Aufpilzung der stegförmigen Bereiche 6 ausgebildet wird. Die Trokarhülse 1 weist zudem in der Fig. 1 einen Trokarkopf 12 auf, welcher beispielsweise ein gasdichtes Ventil 13 und zumindest eine Rastnase 15 umfasst.

In der Fig. 2 ist eine erfindungsgemäße Trokarhülse 1 mit einem eingeführten Trokardorn 2 dargestellt. Dabei ist erkennbar, dass die stegförmigen Bereiche 6 in einer gekrümmten Position, also noch im Ausgangszustand sind.

Im Inneren der Trokarhülse 1 ist ein Trokardorn 2 angeordnet, welcher ebenfalls einen ersten und einen zweiten Bereich 8,9 aufweist. Der erste Bereich 8 ist dabei konisch spitz zulaufend ausgebildet und weist eine Spitze 10 auf, welche zum Einstechen ausgebildet ist.

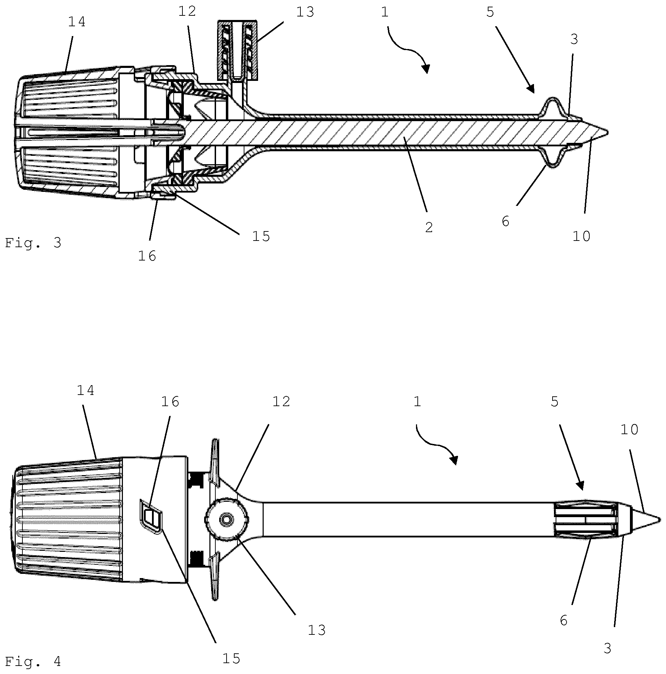

Die Fig. 3 zeigt eine schematische Querschnittsdarstellung der Fig. 2. Dabei ist deutlich der Trokardorn 2 im Inneren der Trokarhülse 1 erkennbar. Zudem zeigt die Fig. 3, dass die Rastnase 15, welche am Trokarkopf 12 angeordnet ist, mit einer Rastnut 16, welche im Inneren des Trokardorngriffs 14 angeordnet ist, im Eingriff steht. Durch eine Drehbewegung des Trokardorngriffs 14 wird die Rastnase 15 in die Rastnut 16 eingerastet, wobei der Trokardorn 2 in Richtung erster Bereich 3 der Trokarhülse 1 bewegt wird. Im Ergebnis kommt es zu einer Streckung der stegförmigen Bereiche 6, wie dies in Fig. 4 abgebildet ist. Durch die halbschalenförmige Ausgestaltung der stegförmigen Bereiche 6, können diese sich bei Streckung in Ausnehmungen 11 des Trokardorns 2 anordnen, sodass eine im Wesentlichen gleichförmige Oberfläche ausgebildet wird. Diese ist insbesondere vor dem Hintergrund der Minimierung von Randverletzungen wichtig. Durch die Verrastung von Rastnase 15 und Rastnut 16 ist eine gleichbleibende Streckung der stegförmigen Bereiche 6 gewährleistet. Im gestreckten Zustand wird der Trokar im Operationsbereich eingestochen und anschließend die Verrastung von Rastnase 15 und Rastnut 16 durch Drehen in entgegengesetzter Richtung gelöst, sodass der Trokardorn 2 aus der Trokarhülse 1 herausgezogen werden kann. Durch das Lösen der Verrastung von Ratsnase 15 und Ratsnut 16 wird die Streckung der stegförmigen Bereiche 6 aufgehoben. Aufgrund der Ausgestaltung der stegförmigen Bereiche 6 aus einem Formgedächtnismaterial nehmen diese nunmehr wieder ihren Ausgangszustand, wodurch es zu einer Aufpilzung kommt. Durch diese Aufpilzung, welche im Lumen erfolgt, wird ein Herausrutschen der Trokarhülse 1 verhindert.

Zum Entfernen der Trokarhülse nach Beendigung der Operation, wird der Trokardorn 2 ggf. mit abgerundeter oder stumpfer Spitze in die Trokarhülse 1 eingeführt und durch Drehung am Trokardorngriff 14 wiederum eine Verrastung von Rastnase 15 und Rastnut 16 bewirkt. Dadurch erfolgt erneut eine Streckung der stegförmigen Bereiche 6, wodurch die Aufpilzung aufgehoben wird und die Trokarhülse 1 nunmehr entfernt werden kann.

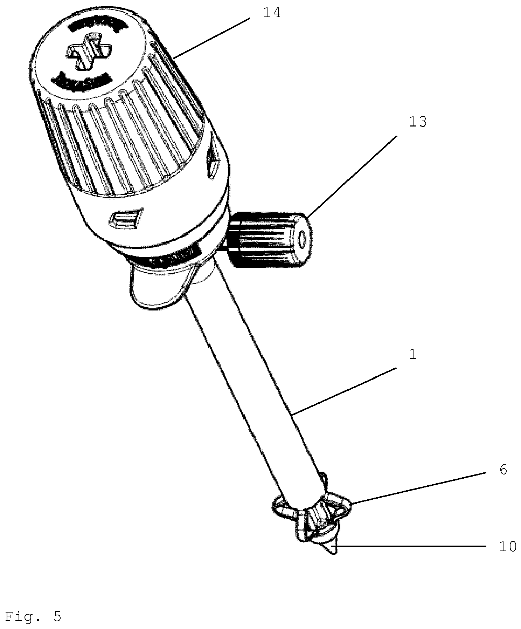

In der Fig. 5 ist noch einmal schematisch eine Trokarhülse 1 mit eingeführtem Trokardorn 2 dargestellt, wobei sich die stegförmigen Bereiche 6 deutlich sichtbar im Ausgangszustand befinden und daher aufgepilzt sind.

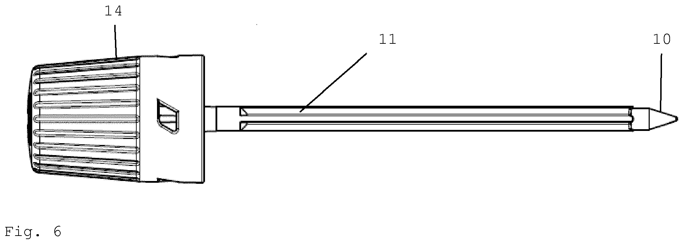

In der Fig. 6 ist ein Trokardorn 2 dargestellt, welcher einen ersten und einen zweiten Bereich 8,9 aufweist. Die Trokardornspitze 10 ist konisch spitz ausgebildet, um ein einfaches Einstechen im Operationsbereich zu gewährleisten. Zudem weist der Trokardorn 2 Ausnehmungen 11 auf, welche entlang der Längsachse des Trokardorn 2 angeordnet sind. Diese Ausnehmungen 11 sind dabei so ausgestaltet, um die Innenseite der stegförmigen Bereiche 6 aufzunehmen, sodass eine im Wesentlichen geschlossene Oberfläche der Trokarhülse 1 ausgebildet wird.

In einem weiteren nicht näher dargestellten Ausführungsbeispiel erfolgt die Anordnung des Trokardorns 2 in der Trokarhülse 1 mittels einer Klemmverbindung.

Am Beispiel einer Intervention wird das Einsatzfeld der neuen Erfindung erklärt. Das intraabdominale Lumen ist mittels einer speziellen Verresnadel und dem Überblähen des Abdomens mit vorgewärmtem CO2 sowie eines speziellen Trokars, wie etwa eines Kameratrokars, über den Bauchnabel leicht zugänglich. Danach werden unter Sicht die erfindungsgemäßen Trokarhülsen bzw. Trokare, sogenannte Arbeitstrokare, platziert. Dies können je nach Operation und Ausführung 1-4 Stück sein, in der Regel jedoch 2 Stück. Über diese kann das/die eigentliche(n) Operationsinstrument(e) eingeführt werden. Nach Beendigung der intraabdominalen Prozedur wird das/die Instrument(e) entfernt und es erfolgt ggf. der kutane Wundverschluss.

### Bezugszeichenliste

- 1: Trokarhülse
- 2: Trokardorn
- 3: erster Bereich der Trokarhülse
- 4: zweiter Bereich der Trokarhülse
- 5: Haltemittel
- 6: stegförmiger Bereich
- 7: Stirnseite des ersten Bereichs der Trokarhülse
- 8: konisch spitz zulaufender erster Bereich des Trokardorns
- 9: zweiter Bereich des Trokardorns
- 10: Spitze des Trokardorns
- 11: Ausnehmungen
- 12: Trokarkopf
- 13: gasdichtes Ventil
- 14: Trokardorngriff
- 15: Rastnase
- 16: Rastnut

## Patentansprüche

1. Trokar umfassend eine Trokarhülse (1) sowie einen Trokarkopf (12), wobei die Trokarhülse (1) einen ersten und einen zweiten Bereich (3,4) umfasst, wobei
- der erste Bereich (3) zur Positionierung innerhalb eines Körpers ist und ein Haltemittel (5) zur Vermeidung des Herausrutschens der Trokarhülse (1) aus dem Körper aufweist, wobei das Haltemittel (5) stegförmig ausgebildete Bereiche (6) aufweist, welche durch Öffnungen (11) in der Trokarhülse (1) voneinander beabstandet die stegförmigen Bereiche (6) aus einem Formgedächtnismaterial ausgebildet sind, wobei die stegförmigen Bereiche (6) einen gebeugten Ausgangszustand aufweisen, wodurch die stegförmigen Bereiche (6) einen aufgepilzten Zustand aufweisen, und
- der zweite Bereich (4) zur Aufnahme eines Trokardorns (2) ausgebildet ist und wobei die Trokarhülse (1) im Inneren zumindest ein Mittel zur lösbaren Anordnung (15) eines Trokardorns (2) aufweist, wobei das im Inneren der Trokarhülse (1) angeordnete Mittel zur lösbaren Anordnung (15) derart ausgebildet ist, um mit einem am Trokardorn (2) angeordneten Mittel zur lösbaren Anordnung (16) in Eingriff zu gelangen, **dadurch gekennzeichnet, dass** die Trokarhülse (1) einstückig ausgebildet ist.

2. Trokar nach Anspruch 1, wobei die stegförmigen Bereiche (6) halbschalenförmig ausgebildet sind.

3. Trokar nach einem der vorhergehenden Ansprüche, wobei die Trokarhülse (1) außen Markierungen zum Ablesen seiner eingeführten Länge aufweist.

4. Trokar nach einem der vorhergehenden Ansprüche, wobei die Trokarhülse (1) einen Innendurchmesser von 3 - 15 mm, über einen zumindest wesentlichen Teil der Gesamtlänge eine Wandstärke von ca. 0,2 - 2 mm und eine Gesamtlänge von 70 - 230 mm aufweist.

5. Trokar nach einem der vorhergehenden Ansprüche, wobei die Trokarhülse (1) eine Rastnase (15) zur Anordnung des Trokardorns (2) aufweist.

6. Trokar nach einem der vorhergehenden Ansprüche, wobei der Trokar einstückig ausgeführt ist.

7. Trokar nach einem der Ansprüche 1 bis 5, wobei der Trokar zweistückig ausgeführt ist, wobei die Trokarhülse (1) und der Trokarkopf (12) lösbar miteinander verbunden sind.

8. Trokar nach einem der vorhergehenden Ansprüche, wobei der Trokarkopf (12) mindestens ein gasdichtes Ventil und/oder mindestens einen Ein- oder Auslass für Gase und/oder Flüssigkeiten umfasst.

9. System umfassend einen Trokar nach einem der Ansprüche 1 bis 8 sowie zumindest einen Trokardorn (2) mit einem ersten und einen zweiten Bereich (8,9), wobei der erste Bereich (8) eine Stirnseite (10) aufweist, wobei der Trokardorn (2) Ausnehmungen (11) aufweist, welche zur Aufnahme der stegförmigen Bereiche (6) einer Trokarhülse (1) ausgebildet sind, wobei der Trokardorn (2) einstückig ausgebildet ist.

10. Verfahren zum Spannen stegförmiger Bereiche (6) einer Trokarhülse (1) eines Trokars nach einem der Ansprüche 1 bis 8 außerhalb des menschlichen oder tierischen Körpers umfassend die Schritte:
- Einführen eines Trokardorns (2) in eine Trokarhülse (1), wobei die Stirnseite des Trokardorns (10) im Inneren an eine Stirnseite der Trokarhülse (5) anschlägt,
- Anordnen des Trokardorns (2) in der Trokarhülse (1) durch zumindest eine lösbare Anordnung, wobei ein im Inneren der Trokarhülse (1) angeordnetes Mittel zur lösbaren Anordnung (15) mit einem am Trokardorn (2) angeordneten Mittel zur lösbaren Anordnung (16) in Eingriff gelangt, sodass der Trokardorn (2) in distaler Richtung bewegt wird, sodass die stegförmigen Bereiche (6) durch die distale Bewegung des Trokardorns (2) von einem gebeugten Ausgangszustand in eine gestreckte Position überführt werden, wodurch die Trokarhülse (1) eine im Wesentlichen geschlossene Oberfläche aufweist.

11. Verfahren nach Anspruch 10, wobei die lösbare Anordnung durch eine formschlüssige Verbindung erfolgt.

12. Verfahren nach einem der Ansprüche 10 oder 11, wobei die lösbare Anordnung durch Verrastung von Trokardorn (2) und Trokarhülse (1) durch einen Rastmechanismus erfolgt.

13. Verfahren nach Anspruch 12, wobei die Verrastung von Rastnut (15) und Rastnase (16) durch Verdrehen des Trokardorns (2) in der Trokarhülse (1) erfolgt.

## Claims

1. Trocar comprising a trocar sleeve (1) and a trocar head (12), the trocar sleeve (1) comprising a first and a second region (3, 4),
- the first region (3) being for positioning within a body and having a holding means (5) to prevent the trocar sleeve (1) slipping out from the body, the holding means (5) having strip-like regions (6) which are spaced apart from each other by openings (11) in the trocar sleeve (1) and which strip-like regions (6) are formed of a shape memory material, the strip-like regions (6) having a bent initial state, whereby the strip-like regions (6) have an expanded state, and
- the second region (4) being formed to receive a trocar shaft (2) and the trocar sleeve (1) having inside at least one means for detachable assembly (15) of a trocar shaft (2), the means for detachable assembly (15) arranged inside the trocar sleeve (1) being formed in such a way as to engage with a means for detachable assembly (16) arranged on the trocar shaft (2), **characterised in that** the trocar sleeve (1) is integrally formed.

2. Trocar according to claim 1, wherein the strip-like regions (6) are half-shell-shaped.

3. Trocar according to either of the preceding claims, wherein the trocar sleeve (1) has outer markings for giving readings on its inserted length.

4. Trocar according to any of the preceding claims, wherein the trocar sleeve (1) has an inner diameter of 3 - 15 mm, a wall thickness of approximately 0.2 - 2 mm over an at least substantial part of the total length, and a total length of 70 - 230 mm.

5. Trocar according to any of the preceding claims, wherein the trocar sleeve (1) has a locking lug (15) for the arrangement of the trocar shaft (2).

6. Trocar according to any of the preceding claims, wherein the trocar is integrally formed.

7. Trocar according to any of the claims 1 to 5, wherein the trocar is formed of two pieces, wherein the trocar sleeve (1) and the trocar head (12) are detachably interconnected.

8. Trocar according to any of the preceding claims, wherein the trocar head (12) comprises at least one gastight valve and/or at least one inlet or outlet for gases and/or liquids.

9. System comprising a trocar according to any of claims 1 to 8 and at least one trocar shaft (2) having a first and a second region (8, 9), wherein the first region (8) has an end face (10), wherein the trocar shaft (2) has recesses (11) which are formed to receive the strip-like regions (6) of a trocar sleeve (1), wherein the trocar shaft (2) is integrally formed.

10. Method for tensioning strip-like regions (6) of a trocar sleeve (1) of a trocar according to any of claims 1 to 8 outside the human or animal body, comprising the steps of:
- inserting a trocar shaft (2) into a trocar sleeve (1), wherein the end face of the trocar shaft (10) strikes an end face of the trocar sleeve (5) on the inside,
- arranging the trocar shaft (2) in the trocar sleeve (1) by means of at least one detachable arrangement, wherein a means for detachable assembly (15) arranged inside the trocar sleeve (1) engages with a means for detachable assembly (16) arranged on the trocar shaft (2), such that the trocar shaft (2) is moved in the distal direction, such that the strip-like regions (6) are transferred from a bent initial state to an extended position by the distal movement of the trocar shaft (2), as a result of which the trocar sleeve (1) has a substantially closed surface.

11. Method according to claim 10, wherein the detachable arrangement is achieved by means of a form-fitting connection.

12. Method according to either claim 10 or claim 11, wherein the detachable arrangement is achieved by locking of the trocar shaft (2) and trocar sleeve (1) by means of a locking mechanism.

13. Method according to claim 12, wherein the locking of the locking groove (15) and locking lug (16) is achieved by rotation of the trocar shaft (2) in the trocar sleeve (1).

## Revendications

1. Trocart comprenant un manchon de trocart (1) et une tête de trocart (12), dans lequel le manchon de trocart (1) comprend une première et une seconde zone (3,4), dans lequel
- la première zone (3) est destinée à être positionnée dans un corps et possède un moyen de retenue (5) permettant d'empêcher le manchon de trocart (1) de glisser hors du corps, dans lequel le moyen de retenue (5) présente des zones en forme de nervures (6) qui sont espacées les unes des autres par des ouvertures (11) dans le manchon de trocart (1) et lesdites zones en forme de nervures (6) sont réalisées à partir d'un matériau à mémoire de forme, dans lequel lesdites zones en forme de nervures (6) présentent un état initial diffracté, amenant ainsi les zones en forme de nervures (6) à se présenter dans un état expansé, et
- la seconde zone (4) est conçue pour recevoir un mandrin de trocart (2) et dans lequel le manchon de trocart (1) présente à l'intérieur au moins un moyen d'agencement amovible (15) d'un mandrin de trocart (2), dans lequel ledit moyen d'agencement amovible (15) situé à l'intérieur du manchon de trocart (1) est conçu pour venir en prise avec un moyen d'agencement amovible (16) disposé sur le mandrin de trocart (2), **caractérisé en ce que** le manchon de trocart (1) est réalisé en une seule pièce

2. Trocart selon la revendication 1, dans lequel les zones en forme de nervures (6) sont en demi-coques.

3. Trocart selon l'une des revendications précédentes, dans lequel le manchon de trocart (1) présente des marquages externes pour la lecture de sa longueur introduite.

4. Trocart selon l'une des revendications précédentes, dans lequel le manchon de trocart (1) présente un diamètre intérieur de 3 à 15 mm, une épaisseur de paroi d'environ 0,2 à 2 mm sur au moins une partie importante de la longueur totale et une longueur totale de 70 à 230 mm.

5. Trocart selon l'une des revendications précédentes, dans lequel le manchon de trocart (1) est doté d'un bec d'encliquetage (15) destiné à positionner le mandrin de trocart (2).

6. Trocart selon l'une des revendications précédentes, dans lequel le trocart est réalisé en une seule pièce.

7. Trocart selon l'une des revendications 1 à 5, dans lequel le trocart est réalisé en deux pièces, dans lequel le manchon de trocart (1) et la tête de trocart (12) sont fixés l'un à l'autre de manière amovible.

8. Trocart selon l'une des revendications précédentes, dans lequel la tête de trocart (12) comprend au moins une soupape étanche au gaz et/ou au moins une entrée ou une sortie pour gaz et/ou liquides.

9. Système comprenant un trocart selon l'une des revendications 1 à 8 et au moins un mandrin de trocart (2) doté d'une première et d'une seconde zone (8,9), dans lequel la première zone (8) présente une face frontale (10), dans lequel le mandrin de trocart (2) présente des évidements (11) qui sont conçus pour recevoir les zones en forme de nervures (6) d'un manchon de trocart (1), et dans lequel le mandrin de trocart (2) est réalisé en une seule pièce.

10. Procédé destiné à tendre les zones en forme de nervures (6) d'un manchon de trocart (1) d'un trocart selon l'une des revendications 1 à 8 à l'extérieur du corps humain ou animal, comprenant les étapes suivantes :
- insertion d'un mandrin de trocart (2) dans un manchon de trocart (1), dans lequel la face frontale du mandrin de trocart (10) est en butée intérieure contre une face frontale du manchon de trocart (5),
- agencement du mandrin de trocart (2) dans le manchon de trocart (1) par au moins un agencement amovible, dans lequel un moyen d'agencement amovible (15) disposé à l'intérieur du manchon de trocart (1) vient en prise avec un moyen d'agencement amovible (16) disposé sur le mandrin (2), de sorte que le mandrin de trocart (2) soit déplacé dans une direction distale, de sorte que les zones en forme de nervures (6) soient transférées d'un état initial diffracté à une position allongée par le mouvement distal du mandrin de trocart (2), amenant ainsi le manchon de trocart (1) à présenter une surface sensiblement fermée.

11. Procédé selon la revendication 10, dans lequel l'agencement amovible est réalisé par une liaison de forme.

12. Procédé selon l'une des revendications 10 ou 11, dans lequel l'agencement amovible est réalisé par encliquetage du mandrin de trocart (2) et du manchon de trocart (1) grâce à un mécanisme d'encliquetage.

13. Procédé selon la revendication 12, dans lequel l'encliquetage de la rainure d'encliquetage (15) et du bec d'encliquetage (16) est effectué par rotation du mandrin de trocart (2) dans le mandrin de trocart (1).
